# EUROPÄISCHE PATENTSCHRIFT

(11) **EP 2 603 304 B1**
(45) Veröffentlichungstag und Bekanntmachung des Hinweises auf die Patenterteilung: **09.04.2014**
(21) Anmeldenummer: 11745696.2
(22) Anmeldetag: 11.08.2011
(51) Int. Cl.: B01D 53/02, B01D 53/62, A61M 16/22, A62B 19/00

(54) **VORRICHTUNG UND VERFAHREN ZUR ABREICHERUNG VON SAUREN GASEN AUS GASGEMISCHEN**
DEVICE AND METHOD FOR DEPLETING ACIDIC GASES FROM GAS MIXTURES
DISPOSITIF ET PROCÉDÉ D'APPAUVRISSEMENT DE GAZ ACIDES À PARTIR DE MÉLANGES GAZEUX

(30) Priorität: 11.08.2010 EP 10008378
(43) Veröffentlichungstag der Anmeldung: 19.06.2013
(73) Patentinhaber: Dräger Safety AG & Co. KGaA, 23560 Lübeck (DE)
(72) Erfinder: KOSEGARTEN, Annette, 23628 Krummesse (DE)
(74) Vertreter: Müller Schupfner & Partner
(86) Internationale Anmeldenummer: PCT/EP2011/004045
(87) Internationale Veröffentlichungsnummer: WO 2012/019770

(56) Entgegenhaltungen:
- WO-A1-98/23370
- US-A- 4 492 649
- US-A- 4 826 805

## Beschreibung

Die Erfindung betrifft eine Vorrichtung und ein Verfahren zur Abreicherung von CO₂ aus Atemgas unter Verwendung von Hydroxid-Verbindungen und Feuchtigkeit aufnehmenden Substanzen.

Geschlossene und halbgeschlossene Atemkreisläufe dienen dazu, dem Anwender eine von der Umgebungsluft ganz oder teilweise unabhängige Atmung zu ermöglichen. Dies ist zum Beispiel bei Arbeitskreislaufgeräten der Feuerwehren, für Gruben, aber auch Fluchtgeräten, Tauchgeräten oder U-Booten der Fall. Durch die Führung der Atemluft im Kreislauf kann Atemgas gespart werden. Dies ist insbesondere wichtig für die Anwendung in der Anästhesie. Es ist allerdings erforderlich, das vom Anwender produzierte und ausgeatmete Kohlendioxid abzureichem.

Das Abreichern des Kohlendioxids erfolgt in der Regel durch Alkali- oder Erdalkalihydroxide mit verschiedensten Beimengungen. Zum Beispiel gemäß folgender Reaktionsschemata:

CO₂ + H₂O -> H₂CO₃

H₂CO₃ + 2 NaOH -> Na₂CO₃ + 2 H₂O

Na₂CO₃ + Ca(OH)₂ -> CaCO₃ + 2 NaOH

Ca(OH)₂ + CO₂ -> CaCO₃ + H₂O + Wärme ΔHR = -113 kJ/mol

So eingesetzte Erdalkalihydroxide werden häufig als Atemkalk bezeichnet. Neben Erdalkalhydroxiden ist Wasser Reaktand, welches für eine optimale Reaktionsfähigkeit des CO₂-Absorbers benötigt wird.

Für die Anwendung in der Anästhesie sind bereits verschiedene Absorptionsmaterialien vorgeschlagen worden. Bekannte Absorptionsmaterialien sind häufig Erdalkalichloride (EP 0939671 B1, WO 98/23370), einfache Silikate oder auch Erdalkalisulfate (EP 0939671 B1). Für die Zwecke der vorliegenden Erfindung ist es aber erforderlich, ein höheres Aufnahmevermögen für Kohlendioxid, insbesondere bei Verwendung feuchter Gase, zu erzielen.

Hierbei wurde gefunden dass die Zugabe herkömmlicher Feuchthaltemittel zu Verlusten in der Aufnahmeleistung der Absorber für saure Gase und zu einer Verringerung der Abriebfestigkeit führen kann.

Bei der Lagerung von Atemkalk kann Wasser durch die Verpackung verloren gehen. Dadurch kann die CO₂-Aufnahmeleistung sinken, da das Wasser nicht mehr in ausreichender Menge als Reaktionsvermittler (vergleiche obiges Reaktionsschemata) zur Verfügung steht. Während des Transports in der jeweiligen Verpackung kann es zusätzlich zu Abrieb (Staub) des Atemkalkes kommen. Dieser Abrieb ist unerwünscht, da er sich ohne geeignete Gegenmaßnahmen im Atemkreislauf befindet und aufgenommen werden kann.

Der Erfindung liegt die Aufgabe zugrunde, Absorptionsmaterial für saure Gase zur Verfügung zu stellen, das die Nachteile des Standes der Technik nicht aufweist und insbesondere einerseits Wasser bereitstellt und andererseits Wasser bindet und lagerstabil ist, d.h. die Absorptionsfähigkeit des Absorbers über die Lagerzeit aufrecht erhalten bleibt, ohne zu Verlusten in der CO₂-Aufnahmeleistung zu führen. Eine gleichzeitige Erhöhung der Abriebfestigkeit ist weiterhin wünschenswert.

Gelöst wird diese Aufgabe durch den Gegenstand der unabhängigen Patentansprüche. Bevorzugte Ausführungsformen sind Gegenstand der Unteransprüche oder nachfolgend beschrieben.

Gegenstand der Erfindung ist die gleichzeitige Verwendung zumindest einer Hydroxid-Verbindung als Absorber für das Sauergas eines Gasgemisches und zumindest einer Wasser absorbierenden Substanz in Form eines superabsorbierenden Polymers (SAP) und/oder Vermiculit, wobei Hydroxid-Verbindung und Wasser absorbierende Substanz in Form eines superabsorbierenden Polymers und/oder Vermiculit miteinander im unmittelbaren räumlichen Kontakt stehen. Sauergas ist Kohlendioxid (CO₂). Das Gasgemisch umfasst Atemgas wie es durch einen Ausatemvorgang bereitgestellt wird. Derartiges ausgeatmetes Atemgas besitzt eine Restfeuchte und ist angewärmt.

Das Gasgemisch kann aber auch ein Tauchgas sein, umfassend z.B. ein Gemisch aus:
a) Stickstoff mit einem Volumenanteil < 79 Vol.% und Sauerstoff mit einem Volumenanteil > 21 Vol. %.
b) Sauerstoff, Stickstoff und Helium, z.B. 20 bis 40 Vol.% Sauerstoff, 20 bis 40 Vol.% Helium und 30-50 Vol.% Stickstoff.

Geeignete Hydroxid-Verbindungen sind insbesondere Erdalkalihydroxide zusammen mit Natriumhydroxid wie Calciumhydroxid zusammen mit Natriumhydroxid und ggf. Lithiumhydroxid. Die Alkalihydroxide werden bevorzugt zu geringeren Anteilen (Massenantelle) als die Erdalkalihydroxide eingesetzt.

Die Wasser absorbierenden Substanzen, vorliegend auch Feuchtebindemittel genannt, sind superabsorbierende Polymere (SAP) oder ein Vermiculit (Schichtsilikat). Diese wirken einerseits als Feuchthaltemittel und andererseits weisen diese eine hohe Feuchtebindefähigkeit auf.

Superabsorbierende Polymere im Sinne der vorliegenden Erfindung sind Polymere, die in der Lage sind. ein Vielfaches ihres Eigengewichts an Wasser aufzusaugen. Geeignete Superabsorber sind z.B. Copolymere aus Acrylsäure und Acrylatsalzen (z.B. Natrlumacrylat), hergestellt unter Verwendung von Vernetzungsmitteln (z.B. Core-Cross-Linker). Für die vernetzten Polyacrylsäuren (crosslinked) können unterschiedliche Vernetzungsmittel zum Einsatz kommen. Durch die Vernetzung der Polymerketten wird das Polymerwasserunlöslich. Wasser dringt in den Polymerpartikel ein, so dass dieser aufquillt und das Wasser bindet. Weitere superabsorbierende Polymere sind vernetzte Polysaccharid-Derivate,

Unter superabsorbierenden Polymeren werden im Rahmen dieser Erfindung vernetzte, organische Polymere verstanden, die in Wasser zwar quellbar, aber nicht löslich sind. Sie quellen mit Wasser auf ein Vielfaches ihres Eigengewichtes an. Geeignete superabsorbierende Polymere umfassen chemisch gesehen teilneutralisierte und vernetzte Polyacrylsäuren, (Teil)-Hydrolysate von Stärke-Acrylnitril-Pfropfcopolymeren, (teil-)neutralisierte Stärke-Acrylsäure Pfropfcopolymere, (teil-)verseifte Vinylacetat Acrylsäureester-Copolymere, (teil-)hydrolysierte Acrylnitril-oder Acrylamidcopolymere, vernetzte Produkte solcher Hydrolysate und Polymere aus vernetzten kationischen Monomeren. Im einzelnen können in den vernetzten superabsorbierenden Polymeren folgende Monomere allein oder in Kombination enthalten sein: Acrylsäure, Methacrylsäure, Vinylsulfonsäure, Styrolsulfonsäure, 2-(Meth)acrylamid-2-methylpropansulfonsäure, 2-(Meth)acryloylethansulfonsäure, 2-(Meth)acryloylpropansulfonsäure sowie die Salze der zuvor genannten Säuren. Ferner (Meth)-acrylamid, N-ethyl-(meth)acrylate, N,N-dimethylaminopropyl-(meth)acrylate, N,N-dimethylaminopropyl(meth)acrylamide sowie deren quaternäre Salze und Vinylpyrrolidon. Als Vernetzer eignen sich beispielsweise Allylmethacrylat, Diethylenglykoldiacrylat, ethoxyliertes Trimethylolpropantriacrylat, Ethylenglykoldiglycidylether, Methylenbisacrylamid, Tetraallyloxyethan, Triallylamin und Trimethylolpropantriacrylat. Weitere Angaben über Superabsorber sind dem Buch " Modern Superabsorbent Polymer Technology", herausgegeben von Fredric L. Buchholz und Andrew T. Graham, Verlag Wiley -VCH (1998) zu entnehmen.

Bevorzugt sind superabsorbierende Polymere auf Basis von Polyacrylat, Polymethacrylat sowie entsprechenden Copolymerisaten.

Vermiculit ist ein Schichtsilikat und kann durch die allgemeine Zusammensetzung:

(Mg_{0,5},Ca_{0,5},Na,K)_{0,7}(M_{g},Fe,Al)₃[(OH)₂|(A1,Si)₂Si₂O₁₀] · 4H₂O

beschrieben werden.

Superabsorber können, je nach Formulierung, bis zu dem 1000-fachen ihres Eigengewichtes an Wasser aufnehmen. Vermiculit immerhin noch bis zu dem 20-fachen, während herkömmliche Feuchthaltemittel, wie zum Beispiel Calciumchlorid, nur etwa das Doppelte ihres Eigengewichts an Feuchtigkeit aufnehmen können.

Der Absorber aufweisend die Absorbermasse enthält bezogen auf den Absorber vorzugsweise größer 10 Gew.% , besonders bevorzugt größer 15 Gew.%. und ggf. auch größer 20 Gew.% Wasser. Der Beladungsgrad mit Wasser in Gew.% kann z.B. mittels des durch Trocknung bei erhöhten Temperaturen erhaltenen Differenzwertes bestimmt werden.

Als Absorbermasse wird im Sinne der vorliegenden Erfindung abschließend die Summe der/den Hydroxid-Verbindungen, der/den superabsorbierendem Polymer(en) und dem Vermiculit verstanden. Der Absorber kann im Unterschied auβer der Absorbermasse weitere Bestandteile enthalten.

Nach einer Ausgestaltung machen obige Wasser absorbierenden Substanzen (superabsorbierendes Polymer(en) und/oder Vermiculit aber ohne Wasser) 0,5 bis 15 Gew.%, insbesondere 0,5 bis 5 Gew.%, der Absorbermasse (100 Gew.%) aus. Den Rest bis auf 100 Gew.% bilden die Hydroxid-Verbindung.

Die Erfindung ist weiterhin gekennzeichnet durch den Aufbau des Absorbers. Das Feuchthaltemittel steht im Absorber, z.B. in Form eines Absorberbettes, mit der Hydroxid-Verbindung in Kontakt, vorzugsweise im allseitigen Kontakt:
- alle Bestandteile können gemeinsam in Form eines Pulvers oder Granulates vorliegen (Schüttung).
- Feuchthaltemittel und Hydroxid-Verbindung sind jeweils in Pulver- oder Granulatform in Schichten neben- /aufeinander angeordnet, die z.B. durch ein Gitterstruktur wie ein Vlies voneinander getrennt sind.
- Hydroxid-Verbindung und/oder Wasser absorbierende Substanz sind gemeinsam, ggf. auch in Schichten, auch auf einem Trägermaterial, wie z.B. einem Vlies aufgebracht (geträgert).
- Das Feuchthaltemittel ist im Absorptionsbett um das Hydroxid herum angeordnet, etwa indem die Hydroxid-Verbindung als Granulat vorliegt und die Wasser absorbierende Substanz, insbesondere der polymere Superabsorber, auf das Granulat aufgezogen ist, oder umgekehrt.

Die Hydroxid-Verbindung kann, ggf. mit anderen Substanzen zusammen, als Granulat vorliegen, typischerweise im Korngrößenbereich zwischen 1 bis 5 mm Grundlage der Korngrößenanalyse kann z.B. ein Verfahren nach der US Pharmacopoeia 27 NF22 sein. Die Granulatform kann dabei unregelmäßig sein, aber auch halbkugel- oder kugelförmig oder als Röllchen oder Tablette.

Das Feuchtebindemittel kann auch um das CO₂-Absorptionsmittel herum als Granulat oder auf einem Träger angeordnet sein, wobei das Feuchtebindemittel für die Hydroxid-Verbindung Träger ist oder umgekehrt.

Es ist auch möglich Hydroxid-Verbindung und Feuchtebindemittel gemeinsam zu Pellets zu verarbeiten, um mit diesen eine Schüttung herzustellen, oder beide auf feste Träger aufzuziehen. Solche Träger können eine Gitter-, Waben- oder Netzstruktur aufweisen.

Die Erfindung findet vorrangig Verwendung in Atemkreislaufgeräten und der Anästhesie, kann aber auch in jeder anderen Anwendung verwendet werden, bei der die heutigen Absorptionsmaterialien auf Basis von Hydroxiden Verwendung für Kohlendioxid finden. Es werden hierbei Atemsysteme verwendet, bei denen das Atemgas im Kreislauf zirkuliert und nach Entfernung/Abreicherung des ausgeatmeten Kohlendioxids dem Atmenden wieder zugeführt wird. Beispiel für Atemkreislaufgeräte sind Kreislauftauchgeräte auch "Rebreather" genannt. Verbrauchtes Atemgas wird substituiert, wobei in Anästhesiesystemen volatile Anästhetika zugegeben werden.

Atemkreislaufgeräte können geschlossen oder halb-geschlossen ausgeführt sein. Halb-geschlossene Atemkreislaufgeräte sind solche, bei denen der verbrauchte Sauerstoff unter Zuhilfenahme einer (Misch)gasquelle ersetzt wird. Durch das stetige bzw. verbrauchsabhängige Hinzufügen von Atemgas in den Kreislauf besteht die Notwendigkeit, überschüssiges Atemgas durch ein geeignetes Ventil oder durch Ausatmen über die Maske abzugeben.

Passiv halbgeschlossene Atemkreislaufgeräte sind solche bei denen der verbrauchte Sauerstoff unter Zuhilfenahme einer (Misch)gasquelle ersetzt wird. Dabei wird bei jedem Atemzug ein konstanter Anteil (z.B. 1: 5 bis 1: 20) des Kreislaufvolumens aus dem Gerät entnommen und nach außen abgegeben. Das verminderte Volumen wird dann wieder automatisch (über Ventile) mit Mischgas aufgefüllt.

Der Vorteil der vorliegenden Erfindung beruht darin, dass durch das Feuchtebindemittel das Wasser im Absorptionsmaterial selbst in großer Menge gebunden werden kann und somit für eine Reaktionsvermittlung mit dem sauren Gas und damit einer Verlängerung der Lagerzeit zur Verfügung steht.

Im Falle der Anästhesieanwendung ist zu erwarten, dass auch keine oder eine reduzierte Narkosemittelzersetzung auftritt, da diese durch Austrocknung begünstigt wird und dies durch Zugabe des Feuchtebindemittels unterbunden wird. Außerdem wird weniger Reaktionswasser aus dem Absorptionsbett in den Atemkreislauf entlassen, so dass der Gerätebetrieb weniger beeinflusst wird und sich eine physiologisch angenehme Einatemfeuchte einstellen kann. Als positiver Nebeneffekt tritt zusätzlich auf, dass die Absorbermasse sowieso nach jedem Gebrauch verbraucht ist und gewechselt werden muss, und somit kein zusätzlicher Aufwand für den Anwender durch Leeren von Kondensatfallen oder Ähnlichem erforderlich ist. Durch die Eigenschaft der Superabsorber, Wasserstoffbrücken zu bilden, die dann das Wasser festhalten, erhöht sich auch die mechanische Beständigkeit, da gerade in Verbindung mit Wasser die Vernetzung innerhalb des Absorbers erhöht wird.

Die Erfindung bzw. deren Eigenschaften sind durch die Figuren näher erläutert. Es zeigen:
Fig. 1: Die Abrasionsneigung verschiedener Atemkalke,
Fig. 2: das CO₂-Absorbtionsvermögen verschiedener Atemkalke,
Fig. 3: eine mögliche Anordnung in einer Filterkartusche und
Fig. 4: eine andere mögliche Anordnung in einer Filterkartusche.

Obige Vorteile sind nicht jedem Wasserbindemittel gegeben, wie die folgenden Versuchsbeispiele zeigen:

### Rezepturbeispiel 1:

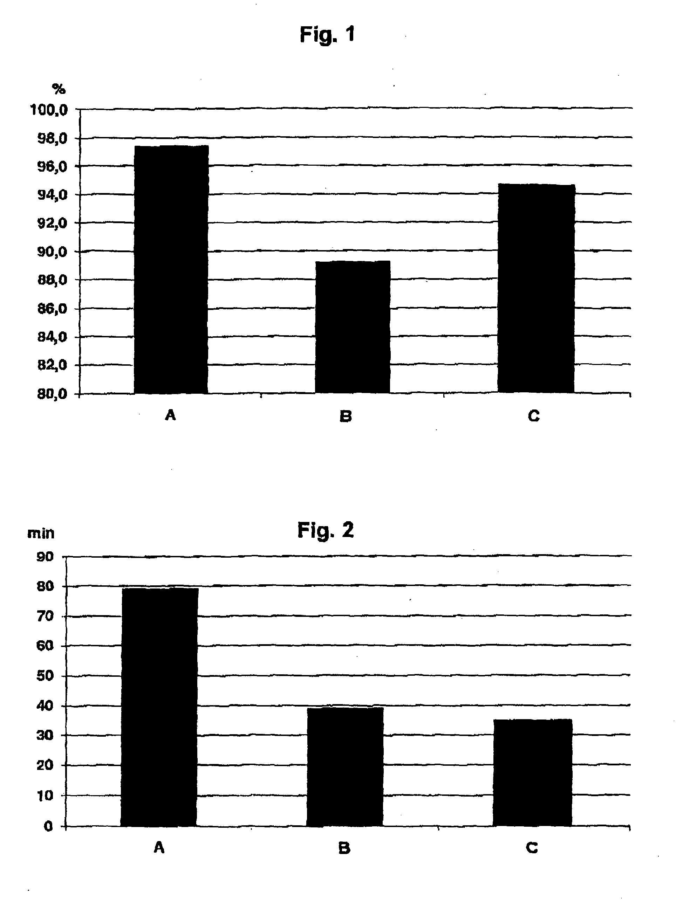

Calciumhydroxid wurde mit 3 Gew.% Natriumhydroxid und 1 Gew.% Superabsorber auf Acrylatbasis und einem Überschuss an Wasser gemischt. Anschlieβend wird die Paste granuliert und auf einen Wassergehalt von ca. 16 % getrocknet. Das erhaltene Produkt ist in Fig. 1 und 2 als Produkt A wiedergegeben.

Die Abriebfestigkeit wurde in einem Friabilator, wie er u.a. für Tablettenprüfungen verwendet wird, über 7 h ermittelt und ist in Fig. 1 gezeigt. Ein herkömmlicher CO₂-Absorber B enthält Calciumchlorid als Feuchtebindemittel, ein anderes herkömmliches Produkt C Silikate.

Im Fall von Produkt B und C ist die Abriebfestigkeit reduziert. Beide Atemkalke mit Bindemittel zeigen eine Verminderung der CO₂-Leistung im STANAG 1411 CO₂-Test (vergleiche Fig. 2). Bei Verwendung von Superabsorber wird die Abriebfestigkeit erhöht bei guten CO₂-Leistungen.

Der Abriebtest wurde so durchgeführt, dass 10 g staubfreier CO₂-Absorber 7 Stunden in einem Friabilator rotiert. Danach wurde über einem 0,42 mm Sieb abgesiebt. Der prozentual errechnete Abrieb (< 0,42 mm) ergab von 100 subtrahiert die Abriebfestigkeit. Der CO₂-Aufnahmetest ist in dem Nato Standard 1411 beschrieben.

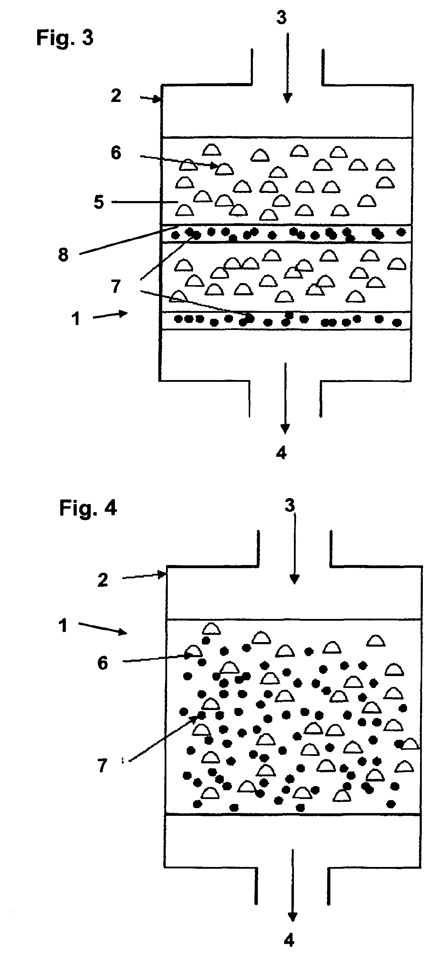

Fig. 4 zeigt schematisch den Aufbau eines CO₂-Absorbers in Form eines Filterelements, wie es in einem Atemsystem eingesetzt werden kann. Das CO₂-Filterelement 1 weist ein Gehäuse 2 mit einem Gaseinlass 3 und einem Gasauslass 4 mit einem als Absorberbett 5 ausgestalteten Absorber auf, versehen mit CO₂-Absorber 6 und Feuchtebindemittel 7 in Form einer Schüttung. In Fig. 3 ist ein Aufbau des Absorbers in Schichten gezeigt, die durch ein Vlies 8 getrennt sind

## Patentansprüche

1. Atemkreislaufgerät zur Abreicherung von Kohlendioxid aus einem Gasgemisch, wobei das Atemkreislaufgerät einen Absorber umfasst, der enthält:
- Kohlendioxid aufnehmende Substanzen, aufweisend oder bestehend aus zumindest einer Hydroxid-Verbindung umfassend zumindest Natriumhydroxid und
- Wasser absorbierende Substanzen aufweisend oder bestehend aus superabsorbierendern Polymer (SAP) und/oder Vermicutit.
wobei
• Hydroxid-Verbindung(en) und superabsorbierendes Polymer (SAP),oder
• Hydroxid-Verbindung(en) und Vermiculit oder
• Hydroxld-Verbindung(en) und superabsorbierendes Polymer und Vermiculit (SAP)
die Absorbermasse bilden und jeweils unmittelbar in Kontakt miteinander stehen und das Gasgemisch Atemgas ist, wie es durch einen Ausatemvorgang bereitgestellt wird, und der Absorber von dem Atemgas durchströmt ist.

2. Atemkreislaufgerät nach Anspruch 1, **dadurch gekennzeichnet, dass** der Absorber unabhängig voneinander
- eine Schichtstruktur hat, umfassend zumindest drei unterschiedliche Schichten der Absorbermasse, wobei zumindest eine Schicht von zwei weiteren anderen Schichten zumindest teilweise umgeben ist,
- eine durchmischte Aufschüttung von Partikeln umfassend die Absorbermasse ist,
- das superabsorbierende Polymer und/oder das Vermiculit und die Hydrorxid-Verbindung auf einem festen oder flexiblen Träger aufgebracht sind,
- das superabsorbierende Polymer und/oder das Vermiculit auf die Hydroxid-Verbindung als Träger aufgebracht ist oder umgekehrt und/oder
- die Hydroxid-Verbindung und das superabsorbierende Polymer und/oder das Vermiculit Jeweils gemeinsam in Partikel eingearbeitet sind.

3. Atemkreislaufgerät nach einem der vorhergehenden Ansprüche, **dadurch gekennzeichnet, dass** die Hydroxid-Verbindung ein Erdalkalihydroxid ist oder umfasst.

4. Atemkreislaufgerät nach einem der vorhergehenden Ansprüche, **dadurch gekennzeichnet, dass** die Hydroxid-Verbindungen ein Erdalkallhydroxid und ein Alkalihydroxid aufweisen, vorzugsweise das Alkalihydroxid zu 0,5 bis 8 Gew.%, bezogen auf die Masse der eingesetzten Hydroxid-Verbindungen.

5. Atemkreislaufgerät nach einem der vorhergehenden Ansprüche, **dadurch gekennzeichnet, dass** die Hydroxid-Verbindung weiterhin Lithiumhydroxid umfasst.

6. Atemkreislaufgerät nach einem der vorhergehenden Ansprüche, **dadurch gekennzeichnet, dass** die Wasser absorbierenden Substanzen 0,5 bis 15 Ges.% der Absorbermasse ausmachen, exkl, ggf. aufgenommenem Wasser.

7. Atemkreislaufgerät nach einem der vorhergehenden Ansprüche, **dadurch gekennzeichnet, dass** der Absorber aufweisend die Absorbermasse bezogen auf den Absorber größer 10 Gew.%, besonders bevorzugt größer 15 Gew%. und ggf. auch größer 20 Gew.% Wasser enthält.

8. Atemkreislaufgerät nach einem der vorhergehenden Ansprüche, **dadurch gekennzeichnet, dass** das Atemgas Anästhesieatemluft umfassend volatile Anästhetika oder ein Tauchgas ist.

9. Atemkreislaufgerät nach einem der vorherigen Ansprüche, wobei der Absorber Teil einer austauschbaren und/oder befüllbaren Kartusche ist.

10. Verfahren zum Entfernen von Kohlendioxid aus einem Gasgemisch unter Verwendung des Atemkreislaufgeräts nach einem der Ansprüche 1 bis 9.

11. Verwendung eines Absorbers enthaltend zumindest folgende Absorbermasse:
- Kohlendioxid aufnehmende Substanzen, aufweisend oder bestehend aus zumindest einer Hydroxid-Verbindung umfassend zumindest Natriumhydroxid und
- Wasser absorbierende Substanzen aufweisend oder bestehend aus su-perabsorbierendem Polymer (SAP) und/oder Vermiculit,
wobei
• Hydroxid-Verbindung(en) und superabsorbierendes Polymer (SAP),oder
• Hydroxid-Verbindung(en) und Vermiculit oder
• Hydroxid-Verbindung(en) und superabsorbierendes Polymer und Vermiculit (SAP)
die Absorbermasse bilden und jeweils unmittelbar in Kontakt miteinander stehen zum Entfernen von Kohlendioxid aus Atemgas, wie es durch einen Ausatemvorgang bereitgestellt wird, wobei das Atemgas den Absorber in einem Atemkreislaufgerät durchströmt, im Kreislauf zirkuliert und nach Entfernung/Abreicherung des ausgeatmeten Kohlendioxids dem Atmenden wieder zugeführt wird.

12. Verwendung nach Anspruch 11, **dadurch gekennzeichnet, dass** der Absorber unabhängig voneinander
- eine Schichtstruktur hat, umfassend zumindest drei unterschiedliche Schichten der Absorbermasse, wobei zumindest eine Schicht von zwei weiteren anderen Schichten zumindest teilweise umgeben ist,
- eine durchmischte Aufschüttung von Partikeln umfassend die Absorbermasse ist,
- das superabsorbierende Polymer und/oder das Vermiculit und die Hydroxid-Verbindung auf einem festen oder flexiblen Träger aufgebracht sind,
- das superabsorbierende Polymer und/oder das Vermiculit auf die Hydroxid-Verbindung als Träger aufgebracht ist oder umgekehrt und/oder
- die Hydroxid-Verbindung und das superabsorbierende Polymer und/oder das Vermiculit jeweils gemeinsam in Partikel eingearbeitet sind.

13. Verwendung nach Anspruch 11, **dadurch gekennzeichnet, dass** die Hydroxid-Verbindungen ein Erdalkalihydroxid und ein Alkalihydroxid aufweisen, vorzugsweise das Alkallhydroxid zu 0,5 bis 8 Gew.%, bezogen auf die Masse der eingesetzten Hydroxid-Verbindungen.

14. Verwendung nach Anspruch 11, **dadurch gekennzeichnet, dass** die Wasser absorbierenden Substanzen 0,5 bis 15 Gew.% der Absorbermasse ausmachen, exkl. ggf. aufgenommenem Wasser.

15. Verwendung nach Anspruch 11, **dadurch gekennzeichnet, dass** das Atemgas Anästhesieatemluft umfassend volatile Anästhetika oder ein Tauchgas ist.

## Claims

1. Circuit breathing apparatus for depleting carbon dioxide from a gas mixture, the Circuit breathing apparatus comprising an absorber which contains:
- carbon dioxide-absorbing substances, including or consisting of at least one hydroxide compound comprising at least sodium hydroxide; and
- water-absorbing substances, including or consisting of superabsorbent polymer (SAP) and/or vermiculite,
wherein
• hydroxide compound(s) and superabsorbent polymer (SAP); or
• hydroxide compound(s) and vermiculite; or
• hydroxide compound(s) and superabsorbent polymer and vermiculite (SAP) form the absorber material and are each directly in contact with each other, and the gas mixture is breathing gas as provided by a breathing out process, and the absorber is flown through by the breathing gas.

2. Circuit breathing apparatus according to claim 1, **characterized in that** the absorber independently
- has a layer structure comprising at least three different layers of the absorber material, wherein at least one layer is surrounded at least partially by two more other layers;
- is a mixed fill of particles comprising the absorber material;
- the superabsorbent polymer and/or vermiculite, and the hydroxide compound are applied to a solid or flexible support;
- the superabsorbent polymer and/or vermiculite is applied to the hydroxide compound as a carrier, or vice versa; and/or
- the hydroxide compound and superabsorbent polymer and/or vermiculite are each jointly incorporated into particles.

3. Circuit breathing apparatus according to one of the preceding claims, **characterized in that** the hydroxide compound is or comprises an alkaline earth metal hydroxide.

4. Circuit breathing apparatus according to one of the preceding claims, **characterized in that** the hydroxide compounds comprise an alkaline earth metal hydroxide and an alkali hydroxide, preferably the alkali hydroxide from 0.5 to 8% by weight, based on the mass of the hydroxide compounds used.

5. Circuit breathing apparatus according to one of the preceding claims, **characterized in that** the hydroxide compound further comprises lithium hydroxide.

6. Circuit breathing apparatus according to one of the preceding claims, **characterized in that** the water-absorbing substances constitute 0.5 to 15% by weight of the absorber material, excluding any optionally absorbed water.

7. Circuit breathing apparatus according to one of the preceding claims, **characterized in that** the absorber including the absorber material, based on the absorber, contains more than 10% by weight, more preferred more than 15% by weight, and optionally also more than 20% by weight of water.

8. Circuit breathing apparatus according to one of the preceding claims, **characterized in that** the breathing gas is anesthesia breathing gas comprising volatile anesthetics, or a diving gas.

9. Circuit breathing apparatus according to one of the preceding claims, wherein the absorber is part of a replaceable and/or refillable cartridge.

10. Process for removing carbon dioxide from a gas mixture using the circuit breathing apparatus according to one of claims 1 to 9.

11. Use of an absorber containing at least the following absorber material:
- carbon dioxide-absorbing substances, including or consisting of at least one hydroxide compound comprising at least sodium hydroxide; and
- water-absorbing substances, including or consisting of superabsorbent polymer (SAP) and/or vermiculite,
wherein
• hydroxide compound(s) and superabsorbent polymer (SAP); or
• hydroxide compound(s) and vermiculite; or
• hydroxide compound(s) and superabsorbent polymer and vermiculite (SAP)
form the absorber material and are each directly in contact with each other, for removing carbon dioxide from breathing gas as provided by an exhaling process, wherein the breathing gas flows through an absorber in a circuit breathing apparatus, is circulated in a circle and is fed back to the breather after removing/depleting the exhaled carbon dioxide.

12. Use according to claim 11, **characterized in that** the absorber independently
- has a layer structure comprising at least three different layers of the absorber material, wherein at least one layer is surrounded at least partially by two more other layers;
- is a mixed fill of particles comprising the absorber material;
- the superabsorbent polymer and/or vermiculite, and the hydroxide compound are applied to a solid or flexible support;
- the superabsorbent polymer and/or vermiculite is applied to the hydroxide compound as a carrier, or vice versa; and/or
- the hydroxide compound and superabsorbent polymer and/or vermiculite are each jointly incorporated into particles.

13. Use according to claim 11, **characterized in that** the hydroxide compounds include an alkaline earth metal hydroxide and an alkali hydroxide, preferably the alkali hydroxide from 0.5 to 8% by weight, based on the mass of the hydroxide compounds used.

14. Use according to claim 11, **characterized in that** the water-absorbing substances constitute 0.5 to 15% by weight of the absorber material, excluding any absorbed water.

15. Use according to claim 11, **characterized in that** the breathing gas is anesthesia breathing gas comprising volatile anesthetics, or a diving gas.

## Revendications

1. Appareil respiratoire pour l'appauvrissement du dioxyde de carbone à partir d'un mélange gazeux, l'appareil respiratoire comprenant un agent absorbant, contenant :
- des substances absorbant le dioxyde de carbone, présentant ou se composant d'au moins un composé d'hydroxyde comprenant au moins de l'hydroxyde de sodium et
- des substances absorbant l'eau présentant ou se composant de polymère superabsorbant (SAP) et/ou de vermiculite,
dans lequel
• le ou les composés d'hydroxyde et le polymère superabsorbant (SAP) ou
• le ou les composés d'hydroxyde et la vermiculite ou
• le ou les composés d'hydroxyde et le polymère superabsorbant et la vermiculite (SAP)
constituent la masse absorbante et sont en contact respectivement direct les uns avec les autres et le mélange gazeux est un gaz respirable, tel qu'il est mis à disposition par un processus d'expiration, et l'agent absorbant est traversé par le gaz respirable.

2. Appareil respiratoire selon la revendication 1, **caractérisé en ce que** l'agent absorbant présente les caractéristiques suivantes, indépendamment les unes des autres :
- il a une structure en couches, comprenant au moins trois couches différentes de masse absorbante, où au moins une couche est au moins partiellement entourée par deux autres couches,
- il est un lit brassé de particules comprenant la masse absorbante,
- le polymère superabsorbant et/ou la vermiculite et le composé d'hydroxyde sont déposés sur un support solide ou flexible,
- le polymère superabsorbant et/ou la vermiculite est/sont déposé(s) sur le composé d'hydroxyde à titre de support ou inversement et/ou
- le composé d'hydroxyde et le polymère superabsorbant et/ou la vermiculite sont incorporés respectivement conjointement dans les particules.

3. Appareil respiratoire selon l'une des revendications précédentes, **caractérisé en ce que** le composé d'hydroxyde est un hydroxyde alcalino-terreux ou comprend ce dernier.

4. Appareil respiratoire selon l'une des revendications précédentes, **caractérisé en ce que** les composés d'hydroxyde présentent un hydroxyde alcalino-terreux et un hydroxyde alcalin, de préférence l'hydroxyde alcalin à un taux de 0,5 à 8 % en poids, par rapport au poids des composés d'hydroxyde mis en oeuvre.

5. Appareil respiratoire selon l'une des revendications précédentes, **caractérisé en ce que** le composé d'hydroxyde comprend en outre de l'hydroxyde de lithium.

6. Appareil respiratoire selon l'une des revendications précédentes, **caractérisé en ce que** les substances absorbant l'eau constituent 0,5 à 15 % en poids de la masse absorbante, à l'exclusion de l'eau éventuellement absorbée.

7. Appareil respiratoire selon l'une des revendications précédentes, **caractérisé en ce que** l'agent absorbant présentant la masse absorbante contient plus de 10 % en poids d'eau par rapport à l'agent absorbant, particulièrement préférentiellement plus de 15 % en poids d'eau et le cas échéant aussi plus de 20 % en poids d'eau.

8. Appareil respiratoire selon l'une des revendications précédentes, **caractérisé en ce que** le gaz respirable est un air respirable anesthésiant comprenant des produits anesthésiants volatils ou un gaz de plongée.

9. Appareil respiratoire selon l'une des revendications précédentes, dans lequel l'agent absorbant fait partie d'une cartouche remplaçable et/ou rechargeable.

10. Procédé d'élimination du dioxyde de carbone à partir d'un mélange gazeux, utilisant l'appareil respiratoire selon l'une des revendications 1 à 9.

11. Utilisation d'un agent absorbant contenant au moins la masse absorbante suivante :
- des substances absorbant le dioxyde de carbone, présentant ou se composant d'au moins un composé d'hydroxyde comprenant au moins de l'hydroxyde de sodium et
- des substances absorbant l'eau présentant ou se composant de polymère superabsorbant (SAP) et/ou de vermiculite,
dans laquelle
• le ou les composés d'hydroxyde et le polymère superabsorbant (SAP) ou
• le ou les composés d'hydroxyde et la vermiculite ou
• le ou les composés d'hydroxyde et le polymère superabsorbant et la vermiculite (SAP)
constituent la masse absorbante et sont en contact respectivement direct les uns avec les autres pour éliminer le dioxyde de carbone du gaz respirable, tel qu'il est mis à disposition par un processus d'expiration, le gaz respirable traversant l'agent absorbant dans un appareil respiratoire, circulant dans le circuit et étant renvoyé à la personne respirant après élimination/appauvrissement du dioxyde de carbone expiré.

12. Utilisation selon la revendication 11, **caractérisée en ce que** l'agent absorbant présente les caractéristiques suivantes, indépendamment les unes des autres :
- il a une structure en couches, comprenant au moins trois couches différentes de masse absorbante, où au moins une couche est au moins partiellement entourée par deux autres couches,
- il est un lit brassé de particules comprenant la masse absorbante,
- le polymère superabsorbant et/ou la vermiculite et le composé d'hydroxyde sont déposés sur un support solide ou flexible,
- le polymère superabsorbant et/ou la vermiculite est/sont déposé(s) sur le composé d'hydroxyde à titre de support ou inversement et/ou
- le composé d'hydroxyde et le polymère superabsorbant et/ou la vermiculite sont incorporés respectivement conjointement dans les particules.

13. Utilisation selon la revendication 11, **caractérisée en ce que** les composés d'hydroxyde présentent un hydroxyde alcalino-terreux et un hydroxyde alcalin, de préférence l'hydroxyde alcalin à un taux de 0,5 à 8 % en poids, par rapport au poids des composés d'hydroxyde mis en oeuvre.

14. Utilisation selon la revendication 11, **caractérisée en ce que** les substances absorbant l'eau constituent 0,5 à 15 % en poids de la masse absorbante, à l'exclusion de l'eau éventuellement absorbée.

15. Utilisation selon la revendication 11, **caractérisée en ce que** le gaz respirable est un air respirable anesthésiant comprenant des produits anesthésiants volatils ou un gaz de plongée.
